(19) Europäisches Patentamt
European Patent Office
Office européen des brevets

(11) Veröffentlichungsnummer: **0 679 397 A2**

# (12) EUROPÄISCHE PATENTANMELDUNG

(21) Anmeldenummer: **95105770.2**

(22) Anmeldetag: **18.04.95**

(51) Int. Cl.[6]: **A61K 31/415**, A61K 31/425, A61K 31/44, A61K 31/495, A61K 31/53

(30) Priorität: **27.04.94 DE 4414569**

(43) Veröffentlichungstag der Anmeldung:
**02.11.95 Patentblatt 95/44**

(84) Benannte Vertragsstaaten:
**AT BE CH DE DK ES FR GB GR IE IT LI LU MC NL PT SE**

(71) Anmelder: **BAYER AG**
**D-51368 Leverkusen (DE)**

(72) Erfinder: **Krüger, Bernd-Wieland, Dr.**
**Am Vorend 52**
**D-51467 Bergisch Gladbach (DE)**
Erfinder: **Maurer, Fritz, Dr.**
**Nakakuki 5-13-9**
**Oyama-shi,**
**Tochigi 323 (JP)**
Erfinder: **Methfessel, Christoph, Dr.**
**Wuppertaler Strasse 62**
**D-45549 Sprockhövel (DE)**
Erfinder: **Tietjen, Klaus, Dr.**
**Am Alten Broich 98**
**D-40764 Langenfeld (DE)**
Erfinder: **Maelicke, Alfred, Professor Dr.**
**Hinter der Hecke 1**
**D-55268 Niederholm (DE)**
Erfinder: **Schmidt, Bernard, Dr.**
**In der Fuhr 8**
**D-51789 Lindlar (DE)**
Erfinder: **Shiokawa, Kozo**
**2-23-30 Shukugawara,**
**Tama-Ku**
**Kawasaki-shi,**
**Kanagawa (JP)**

## (54) Verwendung von substituierten Aminen zur Behandlung von Hirnleistungsstörungen.

(57) Die Erfindung betrifft die Verwendung von substituierten Aminen zur Behandlung sowie Prävention von Hirnleistungsstörungen. Die substituierten Amine eignen sich aufgrund ihrer Eigenschaften als agonistische oder antagonistische Liganden für nikotinärge Rezeptoren insbesondere zur Behandlung und Prävention von seniler und präseniler Demenz, Demenz des Alzheimertyps sowie von Depressionen.

EP 0 679 397 A2

Die Erfindung betrifft die Verwendung von agonistisch oder antagonistisch auf nicotinerge Rezeptoren wirkende substituierten Aminen zur Behandlung sowie Prävention von Hirnleistungsstörungen, entsprechende pharmazeutische Mittel und deren Herstellung.

Es ist bekannt, daß der Neurotransmitter Acetylcholin eine wichtige Rolle bei der Regulation von Lern- und Gedächtnisprozessen im Hirn spielt (R.T. Bartus et al., Science 217: 408, 1982; D. Collerton, Neuroscience 19: 1, 1986). Kognitive Hirnleistungsstörungen, wie sie zum Beispiel im Alter oder bei primär degerativen Erkrankungen auftreten, gehen mit einer Abnahme der cholinergen Neurotransmission einher (P.T. Francis et al., New England J. Med. 313: 7, 1985). Besonders betroffen ist dabei die Funktion nikotinischer Acetylcholinrezeptoren im Hirn (K.K. Kellar, Brain Res. 436: 62, 1987; A. Nordberg et al., Neurosci. Letters 86: 317, 1988; E. Giacobini, J. Neurosci. Res. 27: 548, 1990; A. Nordberg et al., Neurobiol. Aging 13: 747, 1992). Dieses Defizit beschränkt sich dabei auf die präsynaptischen Nikotin-Rezeptoren, während die Expression postsynaptischer Rezeptoren unbeeinflußt bleibt (D.D. Flynn und D.C. Mash, J. Neurochemistry 47: 1948, 1986).

Nach Gabe von Nikotin oder selektiven Nikotinrezeptoragonisten wurden in Tierversuchen positive Effekte auf Lernen und Gedächtnis gefunden (M.W. Decker et al., Brain Res. 572: 281, 1992; S. Poincheval-Fuhrman und S.J. Sara, Behav. Pharmacology 4: 535, 1993; E.D. Levin et al., Cognitive Brain Res. 1: 137, 1993; M.W. Decker et al., Pharmacol. Biochem. Behav. 45: 571, 1993; A.V. Terry et al., Pharmacol. Biochem. Behav. 45: 925, 1993). Außerdem ist bekannt, daß Nikotin in Ratten die cerebrale Durchblutung fördert (D.G. Liniville et al., J. Pharmacol. Exp. Ther. 267: 440, 1993). Kognitiv stimulierende Wirkungen von Nikotin wurden auch in Klinischen Studien in Alzheimer-Patienten belegt (P.A.. Newhouse et al., Psychopharmacology 95: 171, 1988; B. Sahakian, Br. J. Psychiatry 154: 797, 1989).

Es wird daher vermutet, daß Pharmaka mit spezifischer Wirkung an nikotinischen Rezeptoren geeignet sind zur Behandlung und Prävention von Demenzen im Sinne der Definition des Diagnostic and Statistical Manual III (DSM III-R, American Psychiatric Association, APA Press, Washington D.C., 1987), insbesondere von seniler und präseniler Demenz, Demenz des Alzheimer-Typs, Multiinfarktdemenz, AIDS-bezogener Demenz, Demenz bei Down-Syndrom, Störungen der cholinergen und dopaminergen Neurotransmission wie bei der Parkinsonschen und Huntingtonschen Krankheit, sowie Hirnleistungsstörungen infolge von Infarktgeschehen oder Hirntrauma.

Neben lern- und gedächtnisverbessernden Wirkungen wurden auch anxiolytische Eigenschaften von Nikotinrezeptoragonisten beschreiben (J.D. Brioni et al., Eur. J. Pharmacol. 238: 1, 1993). Zudem potenziert Nikotin nach chronischer Gabe die physiologische Wirkung von Dopamin. Darauf begründet sich die Annahme, daß nikotinische Rezeptoragonisten auch zur Behandlung und Prävention von pathologischen Angstzuständen und Depressionen geeignet sind.

Es wurde nun gefunden, daß substituierte Amine der Formel (I)

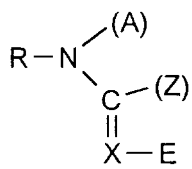

(I)

in welcher

R für Wasserstoff gegebenenfalls substituierte Reste Acyl, Alkyl, Aryl, Aralkyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

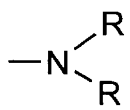

steht oder für eine bifunktionelle Gruppe steht, die mit dem Rest A oder dem Rest X verknüpft ist, nikotinische Rezeptoren des Zentralnervensystems stimulieren und somit zur Behandlung und Prävention von Hirnleistungsstörungen und depressiven Erkrankungen eingesetzt werden können.

Aufgrund der Versuchsergebnisse sind die Verbindungen der Formel (I) geeignet zur Behandlung und Prävention von dementiellen und neurologischen Erkrankungen (z.B. senile und präsenile Demenz, Demenz des Alzheimer-Typs) sowie von Depressionen.

Bevorzugt sind Verbindungen der Formel I, in welcher die Reste folgende Bedeutung haben:

R steht für Wasserstoff sowie für gegebenenfalls substituierte Reste aus der Reihe Acyl, Alkyl, Aryl, Aralkyl, Heteroaryl, Heteroarylalkyl.

Als Acylreste seien genannt Formyl, Alkylcarbonyl, Arylcarbonyl, Alkylsulfonyl, Arylsulfonyl, (Alkyl-)-(Aryl)-phosphoryl, die ihrerseits substituiert sein können.

Als Alkyl sei genannt $C_{1-10}$-Alkyl, insbesondere $C_{1-4}$-Alkyl, im einzelnen Methyl, Ethyl, i-Propyl, sec.- oder t.-Butyl, die ihrerseits substituiert sein können.

Als Aryl sei genannt Phenyl, Naphthyl, insbesondere Phenyl.

Als Aralkyl sei genannt Phenylmethyl, Phenethyl.

Als Heteroaryl sei genannt Heteroaryl mit bis zu 10 Ringatomen und N, O, S insbesondere N als Heteroatomen. Im einzelnen seien genannt Thiophenyl, Furyl, Thiazolyl, Imidazolyl, Pyridyl, Benzthiazolyl.

Als Heteroarylalkyl seien genannt Heteroarylmethyl, Heteroarylethyl mit bis zu 6 Ringatomen und N, O, S, insbesondere N als Heteroatomen.

Als Substituenten seien beispielhaft und vorzugsweise aufgeführt;
Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo ($-SO_3-$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.

A steht für Wasserstoff sowie für gegebenenfalls substituierte Reste aus der Reihe Acyl, Alkyl, Aryl, die bevorzugt die oben angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien.

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.

Als Beispiele für den heterocyclischen Ring seien Pyrrolidin, Piperidin, Piperazin, Hexamethylenimin, Morpholin und N-Methylpiperazin genannt.

E steht für einen elektronentziehenden Rest, wobei insbesondere $NO_2$, CN, Halogenalkylcarbonyl wie 1,5-Halogen-$C_{1-4}$-carbonyl, insbesondere $COCF_3$ genannt seien.

X steht für -CH= oder -N=.

Z steht für gegebenenfalls substituierte Reste Alkyl, -OR, -SR, -NRR, wobei R und die Substituenten bevorzugt die oben angegebene Bedeutung haben.

Z kann gemeinsam mit dem Atom, an welches es gebunden ist und dem Rest

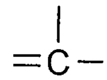

an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.

Als Beispiele für den heterocyclischen Ring seien Pyrrolidin, Piperidin, Piperazin, Hexamethylenimin, Morpholin und N-Methylpiperazin genannt.

Als besonders bevorzugt erfindungsgemäß verwendbare Verbindungen seien Verbindungen der allgemeinen Formeln (II) und (III) genannt:

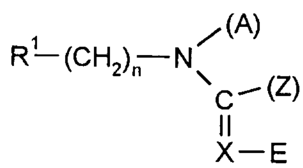

(II)

in welcher

$R^1$ für gegebenenfalls substituiertes Pyridin-3-yl steht, wobei als Substituenten vorzugsweise $C_1$-$C_4$-Alkyl, $C_1$-$C_4$-Alkoxy und Halogen, wie beispielsweise Methyl, Methoxy und Chlor genannt seien;

n für 1 oder 2 steht;

A, Z, X und E die oben angegebene Bedeutung haben;

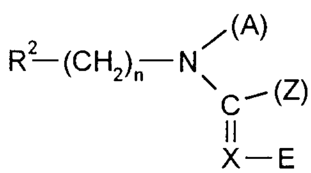

(III)

in welcher

$R^2$ für gegebenenfalls substituiertes 1,3-Thiazol-5-yl steht, wobei als Substituenten vorzugsweise $C_1$-$C_4$-Alkyl, $C_1$-$C_4$-Alkoxy und Halogen, wie beispielsweise Methyl, Methoxy und Chlor genannt seien;

A, Z, X, E und n die oben angegebene Bedeutung haben.

Im einzelnen seien folgende Verbindungen genannt:

3-[(6-Chlor-3-pyridinyl)methyl]-N-nitro-2-thiazolidinimin;
1-[(6-Chlor-3-pyridinyl)methyl]-N-nitro-2-piperidinimin;
1-[(6-Chlor-3-pyridinyl)methyl]-4,5-dihydro-1H-imidazol-cyanamid;
2-Chlor-5-[(2-nitromethylen-1-imidazolidinyl)methyl]-pyridin;
N-[(6-Chlor-3-pyridinyl)methyl]-N,N',N'-trimethyl-N''-nitro-guanidin;
N-[(6-Chlor-3-pyridinyl)methyl]-N'-cyano-N-methyl-ethanimidamid;
1-[(6-Chlor-3-pyridinyl)methyl]-tetrahydro-3,5-dimethyl-N-nitro-1,3,5-triazin-2(1H)-imin;
N-[(6-Chlor-3-pyridinyl)methyl]-N-ethyl-N'-methyl-2-nitro-1,1-ethendiamin;
Tetrahydro-2-nitromethylen-2H-1,3-thiazin;
1-(2-Methylthioethyl)-2-nitromethylen-imidazolidin.

Die erfindungsgemäß verwendbaren Nitromethylene sind bekannt aus den folgenden Publikationen:

Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389;
Deutsche Offenlegungsschriften Nr. 3 639 877, 3 712 307;
Japanische Offenlegungsschriften Nr. 03 220 176, 02 207 083, 63 307 857, 63 287 764, 03 246 283, 04 9371, 03 279 359, 03 255 072;
US-Patentschriften Nr. 5 034 524, 4 948 798, 4 918 086, 5 039 686, 5 034 404;
PCT-Anmeldungen Nr. WO 91/17 659, 91/4965;
Französische Anmeldung Nr. 2 611 114;
Brasilianische Anmeldung Nr. 88 03 621.

Auf die in diesen Publikationen beschriebenen generischen Formeln und Definitionen sowie auf die darin beschriebenen einzelnen Verbindungen wird hiermit ausdrücklich Bezug genommen.

Die Wirkung auf nikotinerge Rezeptoren des Zentralnervensystems wird durch eine für Rezeptorbindungsstudien allgemein übliche Standardmethode (E.C. Hulme, Editor, Receptor-Ligand Interactions, Oxford University Press, 1992, pp 1-458) beschrieben. Hier wurde die Bindung durch Verdrängung von [$^3$H]-Cytisin durch die Wirkstoffe in der Membranpreparation von Rattenhirn gemessen.

Ratten wurden durch Dekapitation getötet und das ganze Hirn sofort danach entnommen und weiterverarbeitet. Alle folgenden Schritte erfolgten unter Kühlung auf 4 °C. Das Gehirn wurde in Saccharose Lösung (110 g Saccharose/l; 20 ml Lösung je 1 g Gehirnmasse) mit Hilfe eines Mixers homogenisiert. Durch Zentrifugation des Homogenats für 10 min bei 1200 g und anschließende Filtration durch 5 Lagen Mull wurden grobe Bestandteile entfernt. Die verbleibende Suspension (im folgenden Homogenat genannt) wurde eingefroren und bis zum Gebrauch bei -20 °C gelagert.

Puffer A für den Testansatz: 100 mM $K_2HPO_4/KH_2PO_4$ pH 7,4, enthaltend 1 g/l Rinderserumalbumin.

Für die Bestimmung der Verdrängung von [$^3$H]-Cytisin durch die Wirkstoffe wurde folgender Ansatz (1 ml Gesamtvolumen) zusammengemischt:
250 $\mu$/l aufgetautes Homogenat, 600 $\mu$/l Puffer, 100 $\mu$/l Wirkstofflösung in Puffer A (enthaltend eine Konzentrationsreihe in Verdünnungsschritten mit dem Faktor 10 zwischen maximal $10^{-2}$ M und minimal $10^{-10}$ M Wirkstoff; sowie 2 $\mu$/l Methanol aus einer konzentrierten Stammlösung der Wirkstoffe), 50 $\mu$/l ($^3$H)-Cytisin Lösung in Puffer A (enthaltend 1,48 pMol ($^3$H)-Cytisin, entsprechend 1,67 KBq, sowie 5 $\mu$/l Ethanol aus einer konzentrierten Stammlösung). In Kontrollen, die kein Homogenat oder keinen Wirkstoff enthielten, wurde das fehlende Volumen durch Puffer A ersetzt.

Die Ansätze wurden für 60 min bei 22 °C in einem Schüttelwasserbad inkubiert. Zum Beenden der Ansätze wurde jeweils 3 ml eiskalter Puffer A zugefügt und die ganze Lösung rasch durch GF/C Glasfaserfilter filtriert. Die Filter waren mit einer 1%igen Lösung von Prosil in Puffer A vorbefeuchtet. Die Filter wurden noch 2 mal mit je 3 ml eiskaltem Puffer A gewaschen. Die Filter wurden anschließend entnommen und die auf den Filtern gebundene Radioaktivität wurde nach Zusatz von 10 ml geeignetem Szintillationscocktail in einem Flüssigkeitsszintillationsgerät bestimmt.

Alle Werte wurden doppelt bestimmt und gemittelt. Aus den Konzentrationsreihen wurde durch eine Logit Transformation oder durch das Vier-Parameter-Logistische-Modell (T. Chard; An Introduction to Radioimmunoassay and Related Techniques; Elsevier, Amsterdam, 1990; pp 163 - 171; statistische Auswertung per Computer) die Konzentration an Wirkstoff berechnet, die zu einer 50%igen Verdrängung des spezifisch gebundenen [$^3$H]-Cytisins führt. Der negative dekadische Logarithmus dieser Konzentration wird im folgenden $pI_{50}$-Wert genannt.

Tabelle 1: Nikotinrezeptor [$^3$H]-Cytisin Verdrängung von Verbindungen der allgemeinen Formel (I)

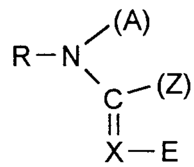

(I)

| Beispiel-Nr. | Struktur | $pI_{50}$-Wert |
|---|---|---|
| 1 | 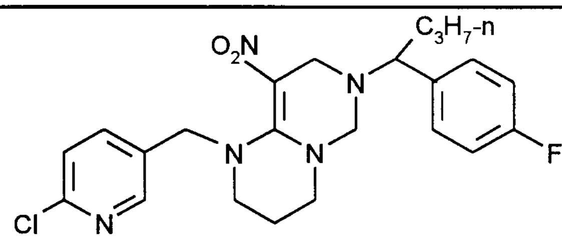 | 6,6 |
| 2 | 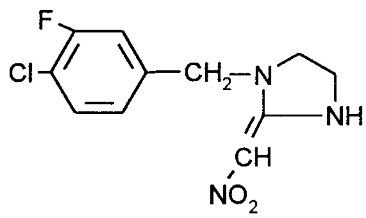 | 5,3 |
| 3 | 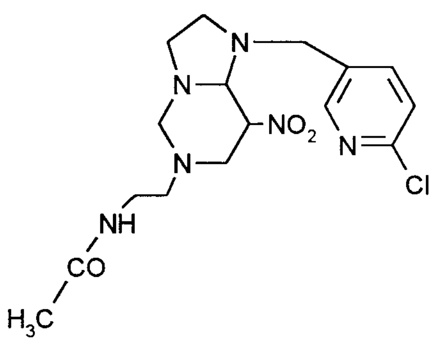 | 6,5 |
| 4 | 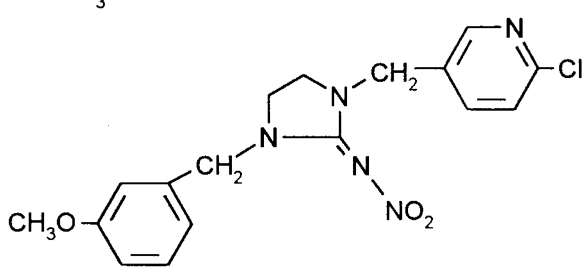 | 5,1 |

(Fortsetzung Tabelle 1)

| Beispiel-Nr. | Struktur | $pI_{50}$-Wert |
|---|---|---|
| 5 | 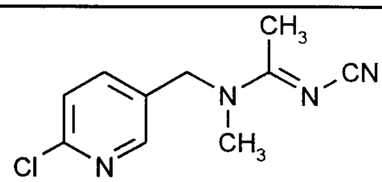 | 6,2 |
| 6 | 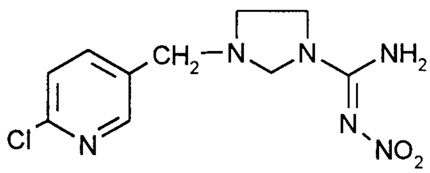 | 4,8 |
| 7 | 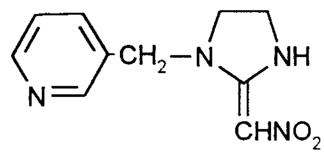 | 5,9 |
| 8 | 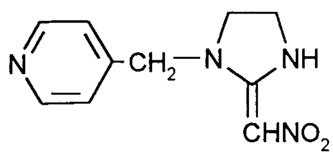 | 4,6 |
| 9 | 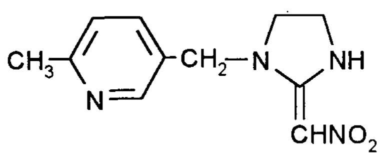 | 6,4 |
| 10 | 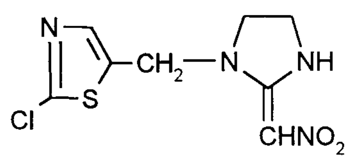 | 6,2 |
| 11 | 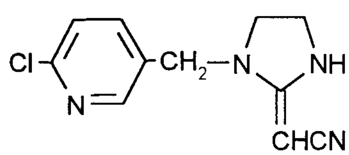 | 5,8 |
| 12 | 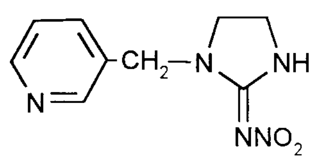 | 5,7 |

(Fortsetzung Tabelle 1)

| Beispiel-Nr. | Struktur | $pI_{50}$-Wert |
|---|---|---|
| 13 | 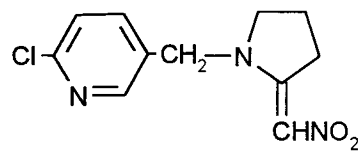 | 6,6 |
| 14 | 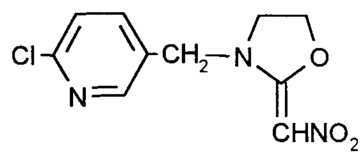 | 6,4 |
| 15 | 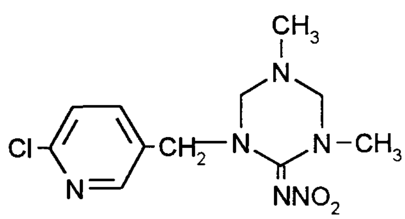 | 5,2 |
| 16 | 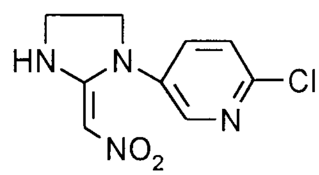 | 4,6 |

Die Wirksamkeit der substituierten Amine auf Lern- und Gedächtnisprozesse wurde im "Active Avoidance Test" untersucht. In diesem aversiv motivierten Test müssen Ratten lernen, auf die andere Seite einer aus zwei Kompartimenten bestehenden Shuttlebox (Coulbourn Instruments) zu wechseln, sobald ein konditionerter Stimulus präsentiert wird. Der konditionierte Stimulus besteht aus dem gleichzeitigen Ertönen eines akustischen Signals und dem Aufleuchten eines Lämpchens. Verläßt die Ratte das Kompartiment der Shuttlebox nicht innerhalb von 6 Sekunden nach dem Beginn des Stimulus, erhält sie einen leichten elektrischen Stromstoß (0,5 mA) über das Bodengitter der Shuttlebox. Die Acquisition des aktiven Vermeidungsverhaltens wird über 50 Konditionierungsversuche erfaßt. Die einzelnen Versuche sind durch variable Pausenintervalle von 20-60 Sekunden voneinander getrennt. Der Anstieg korrekter Vermeidungsaktionen im Verlauf des Trainings dient als Maß für die Lernaktivität der Tiere.

Zur Erfassung von Substanzeffekten auf die Lernkurve (dargestellt durch die 5 Mittelwerte von je 10 sukzessiven Konditionierungsversuchen) werden adulte männliche Ratten 30 Minuten vor dem ersten Test mit Prüfsubstanzen behandelt. Kontrollen erhalten eine entsprechende Menge des Vehikels. Die Testergebnisse zeigen, daß die beschriebenen Verbindungen das Lernverhalten der Tiere positiv beeinflussen können.

Die Wirksamkeit der beschreibenen substituierten Amine bei der Behandlung und Prävention affektiver Erkrankungen wird mit Hilfe des "Rat Forced Swimming Tests" belegt. Dieses Verhaltensmodell wurde

erstmalig von R.D. Porsolt et al. (Nature 266: 730, 1977) beschrieben und ist heute ein allgemein anerkanntes in-vivo Screeningmodell für die Auffindung neuer Antidepressiva. Er beruht auf der Beobachtung, daß Ratten in der ausweglosen Situation in einer unbeweglichen Stellung beharren ("behavioural despair"). In einem Vorversuch werden junge, adulte Ratten (3-4 Monate alt) für 20 min einzeln in Glaszylinder (Höhe 40 cm, Durchmesser 20 cm) gesetzt, welche bis zu einer Höhe von 15 cm mit Wasser gefüllt sind. 24 Stunden nach diesem Vortest werden die Tiere nochmals in die Zylinder überführt und die Dauer der Immobilität über einen Zeitraum von 5 Minuten gemessen.

Die beschriebenen substituierten Amine werden im Zeitintervall zwischen den beiden Schwimmversuchen appliziert. Kontrollen erhalten das Vehikel.

Testergebnisse

| Bsp.-Nr. | $ED_{50}$ [mg/kg p.o.] |
|---|---|
| 3 | 4,1 |
| 10 | 1,5 |
| 12 | 2,5 |

Analog zu in der Literatur beschriebenen, klinisch aktiven Antidepressiva verkürzen die beschriebenen Nitromethylene die Immobilitätsdauer und führen zu einer Verhaltensaktivierung.

Aufgrund der Ergebnisse aus der Verhaltenstestung sind die beschriebenen Verbindungen sowohl zur therapeutischen als auch zur präparativen Behandlung von kognitiven Störungen allgemein, insbesondere von Demenzen des Alzheimer-Typs, als auch von Depressionen geeignet.

Die Erfindung umfaßt ebenfalls pharmazeutische Mittel, die die genannten Verbindungen in einer wirksamen Menge enthalten, deren Herstellung und Verwendung zur Behandlung und Prävention der vorstehend genannten Krankheiten.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös. Die Applikation kann auch transdermal z.B. durch Pflaster erfolgen.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Interwall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

**Patentansprüche**

1. Verwendung von substituierten Aminen der allgemeinen Formel (I)

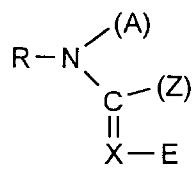

(I)

in welcher

R für Wasserstoff oder gegebenenfalls substituiertes Acyl, Alkyl, Aryl, Aralkyl, Heteroaryl oder Heteroarylalkyl steht;

A für eine monofunktionelle Gruppe aus der Reihe Wasserstoff, Acyl, Alkyl oder Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann und

Z für eine monofunktionelle Gruppe aus der Reihe Alkyl, -O-R, -S-R,

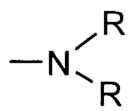

steht oder für eine bifunktionelle Gruppe steht, die mit dem Rest A oder X verknüpft ist,

zur Herstellung von Arzneimitteln zur Behandlung und Prävention von Hirnleistungsstörungen.

2. Verwendung von substituierten Aminen der Formel (I) nach Anspruch 1, wobei

R für Wasserstoff sowie gegebenenfalls substituiertes Acyl, Alkyl, Aryl, Aralkyl, Heteroaryl oder Heteroarylalkyl steht,

A für Wasserstoff sowie gegebenenfalls substituiertes Acyl, Aryl, Alkyl oder für eine gegebenenfalls substituierte Alkylengruppe steht

A und Z gemeinsam mit den Atomen, an welche sie gebunden sind, einen gesättigten oder ungesättigten heterocyclischen Ring bilden können,

E für einen elektronenziehenden Rest steht,

X für die Gruppe -CH= oder -N= steht,

Z für gegebenenfalls substituierte Reste Alkyl, -OR, -SR oder -NRR steht,

und

Z gemeinsam mit dem Atom, an welches es gebunden ist und dem Rest

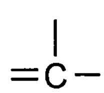

an der Stelle von X einen gesättigten oder ungesättigten heterocyclischen Ring bilden kann

zur Herstellung von Arzneimitteln zur Behandlung und Prävention von Hirnleistungsstörungen.

3. Verwendung von substituierten Aminen der Formel (II)

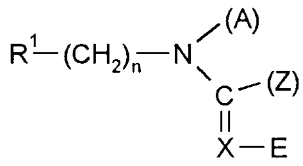

(II)

in welcher

- $R^1$ für gegebenenfalls substituiertes Pyridin-3-yl steht,
- n für 1 oder 2 steht;
- A für eine monofunktionelle Gruppe aus der Reihe Wasserstoff, Acyl, Alkyl, Aryl steht oder für eine bifunktionelle Gruppe steht, die mit dem Rest Z verknüpft ist,
- E für einen elektronenziehenden Rest steht,
- X für die Reste -CH= oder -N= steht, wobei der Rest -CH= anstelle eines Wasserstoffatoms mit dem Rest Z verknüpft sein kann und
- Z für eine monofunktionelle Gruppe aus der Reihe Alkyl, -OR, -SR oder -NRR steht oder für eine bifunktionelle Gruppe steht, die mit dem Rest A oder dem Rest X verknüpft ist,

für die Herstellung von Arzneimitteln zur Behandlung und Prävention von Hirnleistungsstörungen.

**4.** Verwendung von substituierten Aminen der allgemeinen Formel (III)

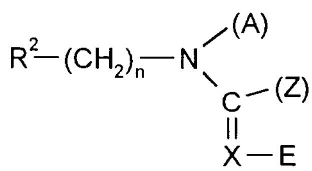

(III)

in welcher

- $R^2$ für gegebenenfalls substituiertes 1,3-Thiazol-5-yl steht,
- A für eine monofunktionelle Gruppe aus der Reihe Wasserstoff, Acyl, Alkyl, Aryl steht oder für eine bifunktionelle Gruppe steht, die mit dem Rest Z verknüpft ist,
- E für einen elektronenziehenden Rest steht,
- X für die Reste -CH= oder -N= steht, wobei der Rest -CH= anstelle eines Wasserstoffatoms mit dem Rest Z verknüpft sein kann und
- Z für eine monofunktionelle Gruppe aus der Reihe Alkyl, -OR, -SR oder -NRR steht oder für eine bifunktionelle Gruppe steht, die mit dem Rest A oder dem Rest X verknüpft ist,

zur Herstellung von Arzneimitteln zur Behandlung und Prävention von Hirnleistungsstörungen.

**5.** Verwendung von substituierten Aminen der Reihe
3-[(6-Chlor-3-pyridinyl)methyl]-N-nitro-2-thiazolidinimin;
1-[(6-Chlor-3-pyridinyl)methyl]-N-nitro-2-piperidinimin;
1-[(6-Chlor-3-pyridinyl)methyl]-4,5-dihydro-1H-imidazol-cyanamid;
2-Chlor-5-[(2-nitromethylen-1-imidazolidinyl)methyl]-pyridin;
N-[(6-Chlor-3-pyridinyl)methyl]-N,N',N'-trimethyl-N''-nitro-guanidin;
N-[(6-Chlor-3-pyridinyl)methyl]-N'-cyano-N-methyl-ethanimidamid;
1-[(6-Chlor-3-pyridinyl)methyl]-tetrahydro-3,5-dimethyl-N-nitro-1,3,5-triazin-2(1H)-imin;
N-[(6-Chlor-3-pyridinyl)methyl]-N-ethyl-N'-methyl-2-nitro-1,1-ethendiamin;
Tetrahydro-2-nitromethylen-2H-1,3-thiazin;
1-(2-Methylthioethyl)-2-nitromethylen-imidazolidin
zur Herstellung von Arzneimitteln zur Behandlung und Prävention von Hirnleistungsstörungen.

**6.** Verwendung von substituierten Aminen nach den Ansprüchen 1 bis 5 zur Herstellung von Arzneimitteln zur Behandlung von Depressionen.

7. Verwendung von substituierten Aminen nach Ansprüchen 1 bis 5 zur Herstellung von Arzneimitteln zur Behandlung und Prävention von dementiellen und neurologischen Erkrankungen.

8. Verwendung von substituierten Aminen nach Ansprüchen 1 bis 5 zur Herstellung von Arzneimitteln zur Behandlung und Prävention seniler und präseniler Demenz.

9. Verwendung von substituierten Aminen nach Ansprüchen 1 bis 5 zur Herstellung von Arzneimitteln zur Behandlung und Prävention von Demenz des Alzheimertyps.